(19) Europäisches Patentamt / European Patent Office / Office européen des brevets

(11) **EP 3 773 788 B1**

(12) **EUROPÄISCHE PATENTSCHRIFT**

(45) Veröffentlichungstag und Bekanntmachung des Hinweises auf die Patenterteilung:
**03.06.2026 Patentblatt 2026/23**

(21) Anmeldenummer: **19716367.8**

(22) Anmeldetag: **04.04.2019**

(51) Internationale Patentklassifikation (IPC):
**A61M 1/16** *(2006.01)* **A61M 1/36** *(2006.01)*

(52) Gemeinsame Patentklassifikation (CPC):
**A61M 1/3639; A61M 1/16; A61M 1/3656;**
A61M 2205/15; A61M 2205/18; A61M 2205/3331;
A61M 2205/702

(86) Internationale Anmeldenummer:
**PCT/EP2019/058494**

(87) Internationale Veröffentlichungsnummer:
**WO 2019/193090 (10.10.2019 Gazette 2019/41)**

(54) **DIALYSEVORRICHTUNG ZUR DURCHFÜHRUNG EINER DIALYSEBEHANDLUNG**

DIALYSIS DEVICE FOR CARRYING OUT A DIALYSIS TREATMENT

DISPOSITIF DE DIALYSE POUR EFFECTUER UN TRAITEMENT PAR DIALYSE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.04.2018 DE 102018107967**

(43) Veröffentlichungstag der Anmeldung:
**17.02.2021 Patentblatt 2021/07**

(73) Patentinhaber: **Fresenius Medical Care Deutschland GmbH**
**61352 Bad Homburg (DE)**

(72) Erfinder:
• **NÜRNBERGER, Thomas**
**97705 Burkardroth (DE)**
• **KELLERMANN, Stefan**
**97711 Maßbach (DE)**

(74) Vertreter: **Nordmeyer, Philipp Werner**
**Maucher Jenkins**
**Patent- und Rechtsanwälte**
**Liebigstraße 39**
**80538 München (DE)**

(56) Entgegenhaltungen:
DE-A1- 102008 015 832    US-B1- 6 601 432
US-B2- 9 925 323

EP 3 773 788 B1

**Beschreibung**

Technisches Gebiet

Die vorliegende Erfindung betrifft eine Dialysevorrichtung zur Durchführung einer Dialysebehandlung und ein Verfahren zur Überwachung eines Betriebszustandes einer Dialysevorrichtung.

Stand der Technik

Verschiedene Verfahren zur Blutbehandlung eines Patienten, insbesondere zur Reinigung und Entwässerung des Blutes eines Patienten, sind bekannt.

Beispielsweise wird bei der Hämodialyse das Blut eines Patienten in einem extrakorporalen Blutkreislauf gereinigt, welcher einen Dialysefilter umfasst. Der Dialysefilter weist eine Blutkammer, durch welche das Blut des Patienten geleitet wird, und eine Dialysatkammer auf. Die Blutkammer und die Dialysatkammer sind durch eine semipermeable Membran, beispielsweise in Form von Hohlfasern, deren Wandung die Membran bildet, voneinander getrennt. Während der Hämodialyse wird die Dialysatkammer von Dialysat durchflossen und die Blutkammer von dem Blut des Patienten durchflossen. Aufgrund eines Konzentrationsgradienten zwischen dem Dialysat und dem Blut des Patienten werden Substanzen aufgrund der Diffusion durch die semipermeable Membran hindurch transportiert.

Weitere Verfahren zur Blutreinigung sind bekannt, wie beispielsweise die Hämofiltration, bei welcher der Substanztransport durch eine semipermeable Membran hindurch aufgrund eines Druckgradienten hervorgerufen wird. Die Hämodiafiltration kombiniert die Hämodialyse und die Hämofiltration.

Der extrakorporale Blutkreislauf umfasst im Wesentlichen ein arterielles Schlauchsystem, durch welches zu reinigendes Blut vom Patienten zum Dialysator gefördert wird, und ein venöses Schlauchsystem, durch welches gereinigtes Blut vom Dialysator dem Patienten wieder zugeführt wird. Das Schlauchsystem kann auch eine Kassette sein, bei der die Schläuche durch beispielsweise zumindest teilweise steife Flüssigkeitsführungen gebildet sind.

Beide Schlauchsysteme können jeweils mit einem Drucksensor der Dialysevorrichtung verbunden sein, welche den Druck vor und nach dem Dialysefilter bestimmen oder den Druck in dem arteriellen Schlauchsystem und in dem venösen Schlauchsystem bestimmen. Dies kann dazu dienen, eine zuverlässige und sichere Dialyse zu gewährleisten. Um eine zuverlässige Drucküberwachung zu gewährleisten, ist es ebenso notwendig, die Funktion der sicherheitsrelevanten Drucksensoren regelmäßig zu überprüfen. Hierfür muss das System im Sinne der Norm IEC 60601-2-16 vor Beginn oder während der Behandlung auf seine Funktionsfähigkeit oder während der Behandlung auf einen Ausfall hin überwacht werden.

Um die Funktion der Drucksensoren zu überprüfen, sind mehrere Verfahren bekannt. Zunächst werden die Drucksensoren bei den bekannten Verfahren im Nullpunkt geprüft, d.h. bei Atmosphärendruck. Um bei einer bekannten Übertragungsfunktion (in der Form $y = mx + t$) des Drucksensors dessen Funktion zu überprüfen, ist jedoch ein weiterer Messpunkt notwendig. Dieser kann durch Anlegen eines zusätzlichen Drucks am Drucksensor erfasst werden, wobei hier eine aufwändige pneumatische Verschlauchung notwendig ist, um den Testdruck der beiden Drucksensoren zu entkoppeln. Eine weitere Möglichkeit besteht darin, mittels einer elektrischen Verstimmung des Sensorsignals den zweiten Messpunkt zu bestimmen. Nachteilig hierbei ist jedoch, dass lediglich die Funktion des Signalpfades nach dem Drucksensor überprüft werden kann, wohingegen die Funktion des Sensors selbst einschließlich seiner Sensormembran nicht getestet werden kann.

Eine weitere Möglichkeit zur Funktionsüberwachung der Drucksensoren besteht darin, für jeden Drucksensor jeweils einen zweiten, redundanten Sensor zu verwenden. Dies erhöht die Gesamtkosten jedoch stark.

Zudem besteht die Möglichkeit, einen zusätzlichen Messpunkt über einen sogenannten Ankoppeltest zu erhalten. Hierbei wird beispielsweise durch Schließen einer Klemme der venöse Druck erhöht und das Erreichen einer Druckschwelle (z. B. venöses Skalenende) geprüft. Der Test kann jedoch erst gestartet werden, wenn ein Schlauchsystem eingelegt und mit einem Fluid gefüllt ist, da bei der Komprimierung von Luft, wie beispielsweise bei einem ungefüllten Schlauchsystem, keine ausreichende Druckänderung aufgebaut werden kann. Außerdem stellt die Durchführung eines Ankoppeltests hohe Anforderung an das Schlauchsystem, weil ein dichtes Abklemmen des Schlauchsystems zu Beginn der Behandlung oder bereits bei der Vorbereitung einer Behandlung ermöglicht werden muss.

Aus der US 9 925 323 B2 ist ein System zur Blutüberwachung in einer extrakorporalen Blutbehandlungsvorrichtung bekannt, welches einen Differenzdrucksensor am extrakorporalen Blutkreislauf zwischen zwei Seiten einer Membranvorrichtung umfasst.

Weiterhin ist aus der US 6,601,432 B1 ist ein Verfahren zum Überprüfen von Differenzdrucksensoren in extrakorporalen Blutbehandlungsvorrichtungen bekannt, wobei Messungen der jeweiligen Sensoren vor der Behandlung gegenüber Luft mit einem vorgegebenen Atmosphärendruck ausgeführt und die jeweiligen Messungswerte miteinander verglichen werden.

Darstellung der Erfindung

Ausgehend von dem bekannten Stand der Technik ist es eine Aufgabe der vorliegenden Erfindung, eine weiter verbesserte Dialysevorrichtung zur Durchführung einer Dialysebehandlung, sowie ein Verfahren zur Überwachung einer Druckmesseinrichtung sowie ein Verfahren zum Ermitteln eines Betriebszustands bereitzustellen.

Die Aufgabe wird durch eine Dialysevorrichtung zur Durchführung einer Dialysebehandlung mit den Merkmalen des Anspruchs 1 gelöst. Vorteilhafte Weiterbildungen ergeben sich aus den Unteransprüchen sowie der vorliegenden Beschreibung und den Figuren.

Entsprechend wird eine Dialysevorrichtung zur Durchführung einer Dialysebehandlung, umfassend ein Flüssigkeitsleitungssystem, welches einen ersten Abschnitt und einen zweiten Abschnitt umfasst, vorgeschlagen. Erfindungsgemäß ist ein Differenzdrucksensor zur Messung eines Differenzdrucks $p_{diffm}$ zwischen einem ersten Druck in dem ersten Abschnitt des Flüssigkeitsleitungssystems und einem zweiten Druck in dem zweiten Abschnitt des Flüssigkeitsleitungssystems vorgesehen, wobei eine Überwachungseinheit vorgesehen ist, die zur Ermittlung eines Betriebszustandes auf Grundlage des gemessenen Differenzdrucks $p_{diffm}$ eingerichtet ist, und weiterhin eine Steuervorrichtung vorgesehen ist, die dazu eingerichtet ist, die Dialysebehandlung entsprechend des ermittelten Betriebszustandes abzubrechen und/oder zu blockieren und/oder eine Anzeigeeinrichtung vorgesehen ist, die zum Ausgeben einer Meldung auf der Grundlage des ermittelten Betriebszustandes eingerichtet ist.

Dadurch, dass ein Differenzdrucksensor zwischen dem ersten Abschnitt und dem zweiten Abschnitt des Flüssigkeitsleitungssystems vorgesehen ist, kann der Differenzdruck auf eine zuverlässige Weise überwacht und/oder die Drucksensoren, die an den einzelnen Abschnitten des Flüssigkeitsleitungssystems angeordnet sind, auf Funktion geprüft werden.

Mittels der Messung des Differenzdrucks kann auch der korrekte Ablauf der Dialysebehandlung überwacht und/oder auf Funktion geprüft werden, wodurch die Sicherheit für den zu behandelnden Patienten erhöht wird. Tritt beispielsweise eine unerwünschte Abweichung des Differenzdrucks während der Dialysebehandlung auf, kann eine Meldung, beispielsweise eine Fehlermeldung, ausgegeben werden und/oder die Dialyse gestoppt werden und/oder der Beginn einer nachfolgenden Behandlung blockiert werden.

Das Flüssigkeitsleitungssystem kann auch zwei oder mehrere Abschnitte aus der Gruppe einer arteriellen Patientenleitung, venösen Patientenleitung, Predialysatordialysatleitung, Postdialysatordiaylsatleitung, Subsituatleitung, Wassereingangsleitung, Konzentratzufuhrleitung aufweisen. Diese Abschnitte können jeweils zum Messen des Drucks in den Leitungen mit einem Drucksensor in Verbindung gebracht sein und es kann jeweils zwischen diesen Abschnitten der Differenzdrucksensor zum Messen eines Differenzdrucks zwischen den Drücken in dem ersten Abschnitt und dem zweiten Abschnitt vorgesehen sein.

Eine Dialysevorrichtung ermöglicht im Allgemeinen die patientenspezifische Entfernung gelöster Substanzen (z. B. Harnstoff, Kreatinin, Vitamin B12 oder β2-Mikroglobulin) sowie gegebenenfalls eines definierten Wasseranteils aus dem Blut bei Nierenersatzbehandlungen. Eine derartige Dialysevorrichtung kann sowohl für eine Hämodialyse als auch eine Hämofiltration oder Hämodiafiltration eingesetzt werden. Ferner kann es sich bei der Dialysevorrichtung um eine Ultrafiltrationsvorrichtung handeln. Dabei wird dem Blut Wasser entzogen ohne eine Reinigung des Blutes. Bei der Dialysevorrichtung kann es sich auch um eine Akutdialysevorrichtung handeln. Bei dieser kann das Dialysat aus einem Beutel in den Dialysekreislauf überführt werden und aus dem Dialysator in einen Beutel überführt werden.

Die Überwachung des Betriebszustandes kann mittels Blut in einem extrakorporalen Kreislauf oder einer anderen Flüssigkeit, insbesondere einer Dialysierflüssigkeit, einer Füll- oder Primingflüssigkeit in dem Flüssigkeitsleitsystem erfolgen. Wenn die Überwachung anhand einer anderen Flüssigkeit als Blut erfolgt, kann diese vor und/oder nach der Behandlung erfolgen, so dass sichergestellt sein kann, dass zumindest vor und/oder nach der Behandlung eine sicher funktionierende Dialysevorrichtung vorliegt.

Der Druck im ersten Abschnitt, wenn es sich bei diesem um die arterielle Patientenleitung im Bereich der Entnahmestelle des Blutes von einem arteriellen Patientenzugang eines Patienten handelt, wird auch als arterieller Druck bezeichnet, der Druck im zweiten Abschnitt, wenn es sich bei diesem um die venösen Patientenleitung im Bereich der Zufuhrstelle des Blutes in einen venösen Patientenzugang des Patienten handelt, wird auch als venöser Druck bezeichnet. Die Einstellung und Erfassung dieser beiden Drücke kann zu verschiedenen Zwecken erforderlich sein. Sie können von Bedeutung für die Sicherheit des Patienten während der Dialysebehandlung sein oder zur Steuerung des Blutflusses und/oder der Dialysebehandlung genutzt werden.

Zwischen dem ersten Abschnitt und dem zweiten Abschnitt kann die Anordnung des genannten Differenzdrucksensors vorgesehen sein.

Unter einem Differenzdrucksensor wird hier unter anderem ein Drucksensor verstanden, der die Differenz zweier Absolutdrücke, d.h. den Differenzdruck, misst. Der Differenzdrucksensor kann beispielsweise aus zwei Messkammern bestehen, die durch eine Membran hermetisch voneinander getrennt sind. Die Auslenkung der Membran ist dann ein Maß für die Größe des Differenzdruckes. Mittels dieses Differenzdrucksensors wird beispielsweise die Druckdifferenz $p_{diffm}$ zwischen dem Druck im ersten Abschnitt $p_1$ und dem Druck im zweiten Abschnitt $p_2$ gemessen. Zwischen den hier

exemplarisch genannten Drücken $p_1$ und $p_2$ besteht folgende Beziehung:

$$p_{diffm} = p_1 - p_2 \qquad (1)$$

Die Überwachungseinheit erfasst die von dem Differenzdrucksensor gemessenen Werte und ermittelt darauf basierend einen Betriebszustand. Dies erfolgt beispielsweise dadurch, dass die gemessenen Werte mit Sollwerten für einen Differenzdruck für bestimmte Betriebszustände verglichen werden oder eine Veränderung des Differenzdrucks über die Zeit hinweg beobachtet wird und beim Auftreten einer abrupten Änderung des Differenzdrucks auf eine Anomalie oder einen unerwünschten Betriebszustand geschlossen wird.

Bei einem Sollwert kann es sich auch um einen Sollverlauf handeln, es muss also nicht ein zeitlich konstanter Wert sein.

Dabei bezeichnen Betriebszustände verschiedene gewünschte oder unerwünschte Zustände des Dialyseverfahrens und/oder der Dialysevorrichtung.

Ein gewünschter Betriebszustand bezeichnet beispielsweise den Fall, in dem der Differenzdruck annähernd konstant ist oder sich periodisch wiederholt oder keine Drift zeigt oder sich eine oder mehrere spezielle Komponenten des Drucksignals über die Zeit nicht ändern und die Dialyse somit effektiv und sicher durchgeführt werden kann. Bei diesen Komponenten kann es sich um die maximale Amplitude des Drucksignals, eine Phase des Drucksignals und/oder eine Frequenzkomponente des Drucksignals, wie sie beispielsweise aus dem periodischen Drucksignal mittels Fouriertransformation erhalten werden kann, handeln.

Ein unerwünschter Zustand liegt beispielsweise dann vor, wenn die venöse Nadel, über welche das gereinigte Blut dem Patienten zugeführt wird, versehentlich von dem venösen Patientenzugang des Patienten gelöst wird (VND, Venous Needle Disconnect) und entsprechend ein Fehlerfall auftritt. In diesem Fall tritt unkontrolliert Blut aus dem Blutrücklauf aus, welches dann dem Patienten nicht mehr zugeführt werden kann, wodurch ein gefährlicher Blutverlust beim Patienten entstehen kann. Dadurch, dass der Kontakt zwischen der venösen Nadel und dem Patienten gelöst ist, fällt der Druck im Blutrücklauf ab, wodurch der Differenzdruck zwischen Blutzulauf und Blutrücklauf fällt (sinkt). Diese Veränderung des Differenzdrucks kann von der Überwachungseinheit als unerwünschter Betriebszustand erkannt werden.

Ein weiterer unerwünschter Betriebszustand kann darin bestehen, dass wenigstens einer der Drucksensoren nicht korrekt funktioniert und dadurch der mit diesem Drucksensor gemessene Druck nicht stimmt.

Wird ein unerwünschter Betriebszustand von der Überwachungseinheit detektiert, kann die Überwachungseinheit sofort das Anhalten des Dialyseprozesses initiieren, um eine Gefährdung des Patienten zu verhindern und/oder eine Meldung beispielsweise ein Alarmsignal oder eine Fehlermeldung ausgeben. Ein Eingriff kann auch darin bestehen, dass die Steuerung der Vorrichtung einen Beginn der Behandlung blockiert. Die Maßnahme als Reaktion eines unerwünschten Betriebszustands kann zeitlich unmittelbar nach Erkennen erfolgen oder zu einem späteren Zeitpunkt. Beispielsweise kann es möglich sein, eine aktuelle Behandlung weiterzuführen und erst nach der Behandlung erfolgt die Maßnahme.

Dadurch, dass mittels der Überwachungseinheit auf Grundlage eines gemessenen Differenzdrucks zwischen dem ersten Abschnitt und dem zweiten Abschnitt verschiedene Betriebszustände einer Dialysevorrichtung ermittelt werden können, wird eine einfache und zuverlässige Lösung zur Überwachung der korrekten Funktion einer Dialysevorrichtung bereitgestellt. Dabei können unerwünschte Betriebszustände schnell und zuverlässig ermittelt werden und sofort oder zu einem vorbestimmten Zeitpunkt oder Ereignis entsprechend in den Dialysevorrichtungsbetriebsvorgang eingegriffen werden, um eine Gefährdung des Patienten, beispielsweise durch erhöhten Blutverlust, zu verhindern.

Erfindungsgemäß sind ein erster Drucksensor zur Messung eines Drucks im ersten Abschnitt und ein zweiter Drucksensor zur Messung eines Drucks im zweiten Abschnitt vorgesehen, und ist die Überwachungseinheit dazu eingerichtet, aus dem ersten gemessenen Druck im ersten Abschnitt und dem zweiten gemessenen Druck im zweiten Abschnitt einen Differenzdruck $p_{diffb}$ zu berechnen.

Gemäß einer Ausführungsform sind ein erster Drucksensor zur Bestimmung eines Drucks im Blutzulauf und/oder ein zweiter Drucksensor zur Bestimmung eines Drucks im Blutrücklauf vorgesehen. Der erste und/oder der zweite Drucksensor können zusätzlich zu dem Differenzdrucksensor vorgesehen sein. Hierdurch können neben dem Differenzdruck zwischen dem Druck im Blutzulauf, d.h. der Druck nahe der Entnahmestelle des zu dialysierenden Blutes, und dem Druck im Blutrücklauf, d.h. der Druck nahe der Zufuhrstelle des dialysierten Blutes in einen Patienten, auch der tatsächliche Druck im Blutzulauf und der tatsächliche Druck im Blutrücklauf unabhängig von dem Differenzdruck bestimmt werden.

Die Überwachungseinheit kann als Betriebszustand eine inkorrekte Funktion des ersten und/oder des zweiten Drucksensors ermitteln.

Die Überwachungseinheit ist dazu eingerichtet, auf Grundlage des berechneten Differenzdrucks $p_{diffb}$ und des gemessenen Differenzdruck $p_{diffm}$ den Betriebszustand zu bestimmen. Dabei kann zur Bestimmung des Betriebszustands die Überwachungseinheit dazu eingerichtet sein, eine Differenz $p_{res}$ zwischen dem berechneten Differenzdruck $p_{diffb}$ und dem gemessenen Differenzdruck $p_{diffm}$ zu bestimmen und die berechnete Differenz $p_{res}$ mit einem Sollwert zu vergleichen.

Gemäß einer weiteren Ausführungsform ist die Überwachungseinheit zum Ermitteln einer korrekten Funktion des ersten und/oder des zweiten Drucksensors auf Grundlage des gemessenen Differenzdrucks und eines aus dem gemessenen Druck im ersten Abschnitt und im zweiten Abschnitt berechneten Differenzdrucks eingerichtet, bevorzugt unter Berücksichtigung eines aus dem gemessenen Druck im ersten Abschnitt und dem gemessenen Druck im zweiten Abschnitt berechneten Differenzdrucks $p_{diffb}$. Dabei kann es sich beispielsweise bei dem ersten Drucksensor um einen an der arteriellen Patientenleitung zum Messen des Drucks angeordneten Drucksensor und bei dem zweiten Drucksensor um einen an der venösen Patientenleitung zum Messen des Drucks angeordneten Drucksensor handeln.

Zur Überwachung der korrekten Funktion der Drucksensoren kann mittels des ersten Drucksensors der absolute Druck im ersten Abschnitt und mittels des zweiten Drucksensors der absolute Druck im zweiten Abschnitt unabhängig von dem Differenzdruck gemessen werden. Aus dem gemessenen Druck im ersten Abschnitt und dem gemessenen Druck im zweiten Abschnitt kann ein Differenzdruck $p_{diffb}$ berechnet werden. Die Überwachungseinheit kann nun dazu eingerichtet sein, den mittels des Differenzdrucksensors gemessenen Differenzdruck $p_{diffm}$ zwischen dem Druck im ersten Abschnitt und dem Druck im zweiten Abschnitt mit dem berechneten Differenzdruck $p_{diffb}$ zu vergleichen. Werden beide Differenzdruckwerte voneinander subtrahiert, ergibt sich ein resultierender Druck $p_{res}$. Bei korrekt messendem ersten und zweiten Drucksensor sowie korrekt messendem Differenzdrucksensor sind die Werte des gemessenen und des berechneten Differenzdrucks gleich, sodass sich ein resultierender Druck von Null ergibt. Zwischen den Drücken, wiederum exemplarisch für die Drücke $p_1$ und p2, besteht folgende Beziehung:

$$p_{res} = (p_1 - p_2) - p_{diffm} \qquad\qquad (2)$$

Da die Messung der Drücke jedoch üblicher Weise toleranzbehaftet ist, kann $p_{res}$ auch ein Wert ungleich Null sein, er ist aber dann ein konstanter oder annähernd konstanter Wert, der sich aus den Messtoleranzen der Drucksensoren ergibt.

Dadurch, dass zusätzlich zu einem ersten und/oder einem zweiten Drucksensor ein Differenzdrucksensor vorgesehen ist, kann die Funktion des ersten und/oder des zweiten Drucksensors auf einfache Weise überprüft werden. Hierdurch wird die Zuverlässigkeit der Dialysevorrichtung und damit die Sicherheit für den Patienten erhöht. Dadurch kann auch eine komplette Funktionskontrolle der Druckmessung, d.h. Widerstandsbrücke, Signalanpassung, ADC-Wandlung und das Einlesen eines Messsignals, nicht notwendig sein. Weiterhin kann auch keine Verstimmung der Drucksensoren notwendig sein, um einen Offset- oder Steilheitsfehler der Drucksensoren zu erkennen, wodurch eine Beeinflussung des Messsignals durch den Verstimmungspfad ausgeschlossen ist.

Ein weiterer Vorteil kann darin bestehen, dass durch die redundante Überwachung kostengünstige Standard-Drucksensoren verwendet werden können und keine speziellen eigensicheren Drucksensoren notwendig sind.

Gemäß einer weiteren Ausführungsform ist das Flüssigkeitsleitungssystem ein extrakorporaler Blutkreislauf mit einem Dialysefilter, wobei der erste Abschnitt einem Blutzulauf und der zweite Abschnitt einem Blutrücklauf entspricht. In dem extrakorporalen Blutkreislauf der Dialysevorrichtung wird das zu reinigende Blut eines Patienten außerhalb des Körpers des Patienten durch die verschiedenen Elemente der Dialysevorrichtung geleitet. Der extrakorporale Blutkreislauf weist einen Blutzulauf (arterielle Patientenleitung) und einen Blutrücklauf (venöse Patientenleitung) auf.

Der Blutzulauf kann mit einer Arterie eines zu behandelnden Patienten verbunden sein, d.h. über den Blutzulauf wird zu reinigendes Blut von dem Patienten in den extrakorporalen Blutkreislauf eingeleitet. Die Förderung des zu reinigenden Bluts kann über eine Pumpe im Blutzulauf erfolgen. Der Blutrücklauf ist mit einer Vene des zu behandelnden Patienten verbunden, d.h. über den Blutrücklauf gelangt gereinigtes Blut aus dem extrakorporalen Blutkreislauf in den Körper des Patienten. In der Regel erfolgt die Blutentnahme und Blutrückgabe aus und in ein extra geformtes Blutgefäß (Shunt) oder ein großes blutführendes Gefäß des Patienten. Zwischen dem Vorlauf und dem Blutrücklauf des extrakorporalen Blutkreislaufs befindet sich ein Dialysefilter. Das zu reinigende Blut wird durch diesen Dialysefilter geleitet, wobei beispielsweise bei der Hämodialyse im Gegenstrom zu dem zu reinigenden Blut ein Dialysat fließt und wobei der Dialysatstrom und der Blutstrom über eine semipermeable Membran voneinander getrennt sind. In dem Dialysefilter werden somit aufgrund von Diffusion unerwünschte Substanzen aus dem Blut entfernt und das Blut mit gewünschten Substanzen angereichert.

Gemäß einer Ausführungsform ist die Überwachungseinheit dazu eingerichtet, mittels des gemessenen Differenzdrucks $p_{diffm}$ eine Nadeldiskonnektion (VND, Venous Needle Disconnect) zu detektieren. Der Begriff Nadeldiskonnektion umfasst dabei auch den Fall, dass sich eine Verbindung in dem Flüssigkeitsleitungssystem löst, beispielsweise eine Luer Verbindung vom Schlauchsystem zur Nadel oder auch die Verbindung zwischen Schlauchsystem und Dialysator.

Die Erkennung einer Nadeldiskonnektion kann umso zuverlässiger erfolgen, je weniger druckbeeinflussende Bauteile vorgesehen sind. Dies ist insbesondere dadurch möglich, dass die Dialysevorrichtung zur Durchführung einer Dialysebehandlung im extrakorporalen Blutkreislauf lediglich eine Blutpumpe, einen Dialysator, einen venöse Kammer mit Gerinselfänger und ein Schlauchsystem, welches die genannten Elemente verbindet, umfasst. Es sind also keine Elemente, welche den Differenzdruck besonders beeinflussen, beziehungsweise Druckschwankungen erzeugen, vorgesehen. Insbesondere umfasst diese Vorrichtung lediglich eine oder mehre Pumpen für den Dialysatfluss und eine Pumpe für den Blutfluss, jedoch keine Pumpe, mit der der Dialysat beziehungsweise das Substituat direkt, d.h. nicht über die Membran des Dialysators, in das Blutschlauchsystem überführt werden kann.

Durch die Verwendung des Differenzdrucksensors und damit von nur einem Druckaufnehmer ergeben sich keine Linearitätfehler und keine Phasenunterschiede wie bei der Bestimmung der Nadeldiskonnektion auf der Grundlage von $p_{diffb}$, für dessen Berechnung das Signal zweier Drucksensoren herangezogen werden muss.

Die oben gestellte Aufgabe wird weiterhin durch ein Verfahren zur Überwachung von Betriebszuständen in einer Dialysevorrichtung mit den Merkmalen des Anspruchs 6 gelöst. Vorteilhafte Weiterbildungen des Verfahrens ergeben sich aus den Unteransprüchen sowie der vorliegenden Beschreibung und den Figuren.

Entsprechend wird ein Verfahren zur Überwachung wenigstens eines Betriebszustands einer Dialysevorrichtung, die ein Flüssigkeitsleitungssystem gefüllt mit einer anderen Flüssigkeit als Blut mit einem ersten Abschnitt und einem zweiten Abschnitt umfasst, vorgeschlagen. Das Verfahren umfasst die Schritte

- Messen eines Differenzdrucks $p_{diffm}$ zwischen einem ersten Druck im ersten Abschnitt und einem zweiten Druck im zweiten Abschnitt mittels eines Differenzdrucksensors,

- Ermitteln des Betriebszustandes auf Grundlage des gemessenen Differenzdrucks $p_{diffm}$, und

- Ausgeben einer Meldung auf Grundlage des ermittelten Betriebszustandes.

Beim Ermitteln einer Abweichung des gemessenen Differenzdrucks $p_{diffm}$ von einem Sollwert und/oder bei Ermitteln einer vorgegebenen Veränderung des Differenzdrucks $p_{diffm}$ während der Dialysebehandlung kann dabei auf einen unerwünschten Betriebszustand geschlossen werden.

Die Abweichung kann sich in einer Abweichung eines rechnerisch verarbeiteten gemessenen Differenzdrucks $p_{diffm}$ ergeben. Die rechnerische Verarbeitung kann beispielsweise eine zeitliche Mittelung und/oder eine Fourieranalyse für eine frequenzaufgelösten Auswertung und/oder eine Subtraktion eines Sollsignalverlaufs aufweisen.

Nach dem Ermitteln eines unerwünschten Betriebszustandes wird eine Meldung auf Grundlage des ermittelten Betriebszustandes ausgegeben, beispielsweise eine Fehlermeldung. Verändert sich der Differenzdruck $p_{diffm}$ beispielsweise aufgrund einer versehentlich gelösten venösen Nadel oder einer defekten Flüssigkeitszufuhr/-abfuhr plötzlich, wird diese Druckveränderung von der Überwachungseinheit zur Ermittlung des Betriebszustands detektiert, welche daraufhin ein sofortiges Anhalten der Dialysebehandlung initiieren kann, um beispielsweise einen gefährlichen Blutverlust des Patienten zu vermeiden.

Erfindungsgemäß umfasst das Verfahren die Schritte

- Messen eines Drucks im ersten Abschnitt mittels eines ersten Drucksensors,

- Messen eines Drucks im zweiten Abschnitt mittels eines zweiten Drucksensors,

- Berechnen eines Differenzdrucks $p_{diffb}$ zwischen dem gemessenen Druck im ersten Abschnitt und dem gemessenen Druck im zweiten Abschnitt.

Das Verfahren wird erfindungsgemäß vor und/oder nach einer Behandlung durchgeführt.

Zwischen dem gemessenen Druck im ersten Abschnitt, dem gemessenen Druck im zweiten Abschnitt und dem berechneten Differenzdruck $p_{diffb}$ besteht folgende Beziehung - hier wieder exemplarisch beschrieben an Hand der Drücke $p_1$ und $p_2$:

$$p_{diffb} = p_1 - p_2 \qquad\qquad (3)$$

Gemäß einer Ausführungsform des Verfahrens umfasst der Betriebszustand eine inkorrekte Funktion des ersten und/oder des zweiten Drucksensors. Hierdurch kann nicht nur der Druck im ersten und/oder im zweiten Abschnitt überwacht werden, sondern zusätzlich die korrekte Funktion des ersten und des zweiten Drucksensors. Damit wird verhindert, dass der Druck im ersten Abschnitt und/oder der Druck im zweiten Abschnitt falsch gemessen werden. Hierdurch wird insgesamt die Sicherheit des Dialyseverfahrens erhöht.

Wird nämlich der Druck im Vorlauf falsch bestimmt beziehungsweise ein Messfehler nicht erkannt, erfolgt möglicherweise eine fehlerhafte Berechnung des dialysierten Blutvolumens. Wird der Druck im Blutrücklauf falsch bestimmt beziehungsweise ein Messfehler nicht erkannt, wird ein Druckabfall, welcher durch eine versehentlich gelöste Verbindung zur venösen Nadel oder durch ein Ablösen der Nadel vom Patienten ausgelöst wird, möglicherweise nicht erkannt. Dies kann einen Blutverlust und damit eine erhebliche Gefährdung des Patienten zur Folge haben.

Gemäß einer Ausführungsform des Verfahrens wird nach Ermittlung einer inkorrekten Funktion eines ersten Drucksensors in einem ersten Abschnitt aus dem gemessenen Differenzdruck $p_{diffm}$ und dem gemessenen Druck in einem zweiten Abschnitt der Druck im ersten Abschnitt bestimmt.

Gemäß einer weiter bevorzugten Ausführungsform des Verfahrens kann nach Ermittlung einer inkorrekten Funktion eines zweiten Drucksensors in einem zweiten Abschnitt aus dem gemessenen Differenzdruck $p_{diffm}$ und dem gemessenen Druck in einem ersten Abschnitt der Druck im zweiten Abschnitt bestimmt werden. Ergibt der Vergleich zwischen dem gemessenen und dem berechneten Differenzdruck, dass einer der beiden Drucksensoren ausgefallen ist, d.h. gar nicht misst, oder ein falsches Messergebnis geliefert hat, kann der fehlende Messwert über folgende Beziehungen berechnet werden - hier wieder exemplarisch beschrieben an Hand der Drücke $p_1$ und $p_2$:

$$p_1 = p_2 - p_{diffm} \qquad (6)$$

$$p_2 = p_1 + p_{diffm} \qquad (7)$$

Dadurch, dass ein fehlender Messwert mittels des gemessenen Differenzdrucks berechnet werden kann, ist es möglich, dass auch bei einem Ausfall oder einer fehlerhaften Messung des ersten und/oder des zweiten Drucksensors das Dialyseverfahren zuverlässig fortgesetzt werden kann.

Gemäß einer Ausführungsform umfasst das Ermitteln des Betriebszustands ein Ermitteln auf der Grundlage des berechneten Differenzdrucks $p_{diffb}$ und des gemessenen Differenzdrucks $p_{diffm}$.

Das Ermitteln kann folgende Schritte umfassen:

- Berechnen eines resultierenden Drucks $p_{res}$ aus der Differenz zwischen dem berechneten und dem gemessenen Differenzdruck $p_{diffm}$ und dem gemessenen Differenzdruck $p_{diffb}$, und

- - Analyse des Verhaltens des Drucks $p_{res}$

Die Analyse kann ein Abgleich mit einem Sollwert und/oder eine Analyse der Veränderung über die Zeit sein.

Der resultierende Druck $p_{res}$ wird folgendermaßen berechnet:

$$p_{res} = p_{diffm} - p_{diffb} \qquad (4)$$

Für eine Überwachung, beispielsweise eine sogenannte T0-Überwachung, d.h. eine Überwachung während der Behandlung oder eine T1-Prüfung, d.h. eine Prüfung außerhalb der Behandlung, auf einen funktionsfähigen ersten und/oder zweiten Drucksensor, gilt folgende Beziehung:

$$\Delta p_{res} = \left| (p_{ven} - p_{art}) - p_{diffm} \right| \qquad (5)$$

Hierbei wird bei einer Überwachung eine gewisse Abweichung $\Delta p_{res}$ des resultierenden Drucks zugelassen, da jeder der Drucksensoren Toleranzen bezüglich der Temperaturdrift, der Steilheit oder des Offsets aufweist. Die zulässige Abweichung kann in einem Speicher der Dialysevorrichtung abgelegt sein und durch Vergleich kann ein Prozessor der Dialysevorrichtung ermitteln, ob die zulässige Abweichung eingehalten und/oder überschritten wird.

Spätestens bei einem nachfolgenden T1-Test kann das von einer Überwachungseinheit erkannte Problem angezeigt werden. Hierauf kann beispielsweise mittels einer Checkliste das aufgetretene Problem spezifiziert werden beziehungsweise auf den entsprechenden Drucksensor eingeschränkt werden.

Es besteht weiterhin die Möglichkeit nach einem erkannten Fehler über ein kurzzeitiges Schließen einer venösen Klemme während der Behandlung zu überprüfen, welcher der beiden Drucksensoren ausgefallen ist.

Gemäß einer Ausführungsform des Verfahrens wird bei einer Abweichung des resultierenden Drucks vom Sollwert aus dem resultierenden Druck $p_{res}$ ein Fehlervolumen kumuliert. Beim Überschreiten eines vorgegebenen maximalen kumulierten Fehlervolumens erfolgt die Meldung und/oder die Dialysebehandlung wird abgebrochen und/oder der Start einer Behandlung wird blockiert.

Gemäß einer bevorzugten Ausführungsform des Verfahrens erfolgt bei einer anhaltenden Abweichung des

resultierenden Drucks $p_{res}$ vom Sollwert bei Erreichen einer Auslöseschwelle eine Meldung.

Durch das beschriebene Überwachungsverfahren ist es möglich, den Druck in dem ersten Abschnitt, beispielsweise im Vorlauf, d.h. den arteriellen Druck, als auch den Druck in dem zweiten Abschnitt, beispielsweise im Blutrücklauf, d.h. den venösen Druck, und deren Differenzdruck zu überwachen. Mittels des Überwachungsverfahrens kann die somit der Verlauf der Behandlung auf einfache Weise überwacht werden und dadurch ein korrekter Ablauf und eine effektive Behandlung des Patienten gewährleistet werden.

In einem Beispiel, welches nicht von der Erfindung beansprucht wird, kann das Flüssigkeitsleitungssystem ein extrakorporaler Blutkreislauf mit einem Dialysefilter sein, wobei der erste Abschnitt einem Blutzulauf und der zweite Abschnitt einem Blutrücklauf entspricht.

In einem weiteren Beispiel, welches nicht von der Erfindung beansprucht wird, kann auf Grundlage des gemessenen Differenzdrucks $p_{diffm}$ eine Nadeldiskonnektion als Betriebszustand erkannt werden.

Das Verfahren kann mit einer zuvor beschriebenen Dialysevorrichtung ausgeführt werden, das heißt, sämtliche für die Dialysevorrichtung beschriebenen Merkmale sind auch für das Verfahren offenbart und umgekehrt.

Kurze Beschreibung der Figuren

Bevorzugte weitere Ausführungsformen der Erfindung werden durch die nachfolgende Beschreibung der Figuren näher erläutert. Dabei zeigen:

Figur 1      eine Dialysevorrichtung mit einem extrakorporalen Blutkreislauf eines Dialyseapparates mit einem Differenzdrucksensor zwischen einem Blutzulauf und einem Blutrücklauf,

Figur 2      ein Diagramm, welches einen gewünschten und einen unerwünschten Betriebszustand der Dialysevorrichtung aus Figur 1 zeigt,

Figur 3      eine Dialysevorrichtung mit einem extrakorporalen Blutkreislauf eines Dialyseapparates mit einem ersten Drucksensor in einem Blutzulauf und einem zweiten Drucksensor in einem Blutrücklauf und einem Differenzdrucksensor zwischen dem Blutzulauf und dem Blutrücklauf,

Figur 4      ein Diagramm, welches einen beispielhaften Druckverlauf der Dialysevorrichtung aus Figur 3 mit korrekt messenden Drucksensoren darstellt,

Figur 5      ein Diagramm, welches einen beispielhaften Druckverlauf der Dialysevorrichtung aus Figur 3 darstellt, wobei ein zweiter Drucksensor im Blutrücklauf falsch misst,

Figur 6      eine Dialysevorrichtung mit einem extrakorporalen Blutkreislauf eines Dialyseapparates, wobei verschiedene Anordnungsmöglichkeiten von Drucksensoren, die den Druck in verschiedenen Abschnitten messen können, veranschaulicht sind,

Figur 7      eine Dialysevorrichtung mit einem extrakorporalen Blutkreislauf eines Dialyseapparates, wobei weitere Anordnungsmöglichkeiten des Differenzdrucksensors veranschaulicht sind,

Figur 8      eine schematische Darstellung eines Verfahrens zur Überwachung eines Betriebszustands einer Dialysevorrichtung auf Grundlage eines gemessenen Differenzdrucks, und

Figur 9      eine schematische Darstellung eines Verfahrens zur Überwachung eines Betriebszustands einer Dialysevorrichtung auf Grundlage eines berechneten und eines gemessenen Differenzdrucks.

Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren mit identischen Bezugzeichen versehen, und auf eine wiederholte Beschreibung dieser Elemente wird teilweise verzichtet, um Redundanzen zu vermeiden.

In der folgenden Beschreibung wird eine Dialysevorrichtung 1 zur Durchführung einer Dialysebehandlung und ein Verfahren zur Überwachung wenigstens eines Betriebszustands der Dialysevorrichtung 1 beschrieben. Die oben bereits allgemein beschriebene Dialysevorrichtung 1 ist in den Figuren sehr schematisch und exemplarisch anhand eines extrakorporalen Blutkreislaufs 10 mit zumindest einem Dialysefilter 5 und einem Flüssigkeitsleitsystem 2 beschrieben.

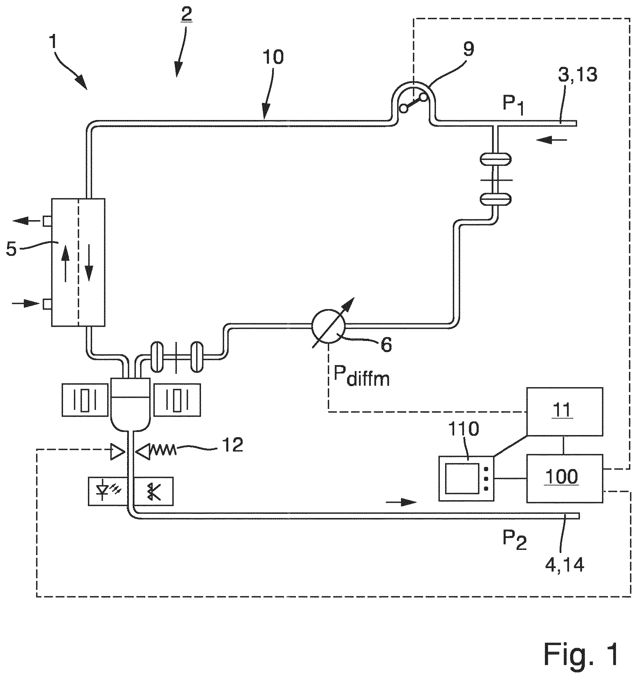

In Figur 1 ist entsprechend schematisch eine Dialysevorrichtung 1 mit einem Flüssigkeitsleitungssystem 2 gezeigt, wobei das Flüssigkeitsleitungssystem 2 ein extrakorporaler Blutkreislauf 10 ist. Dabei entspricht für die weitere Beschreibung ein Blutzulauf 13 einem ersten Abschnitt 3 des Flüssigkeitsleitungssystems 2 und ein Blutrücklauf 14 einem zweiten Abschnitt 4 des Flüssigkeitsleitungssystems 2.

Die Dialysevorrichtung 1 umfasst weiter einen zwischen dem Blutzulauf 13 und dem Blutrücklauf 14 angeordneten Dialysefilter 5. Der Blutzulauf 13, auch arterieller Zulauf genannt, ist über eine hier nicht weiter gezeigte Verbindung mit dem Patienten verbunden, um Blut aus dem Kreislauf des Patienten zu entnehmen. Der Blutrücklauf 14, auch venöser Rücklauf genannt, ist ebenfalls über eine hier nicht weiter gezeigte Verbindung mit dem Patienten verbunden, um das in dem Dialysefilter 5 behandelte Blut wieder in den Kreislauf des Patienten zurückzuführen.

Die Dialysevorrichtung 1 weist weiterhin eine Pumpe 9, hier in Form einer Schlauchpumpe gezeigt, zur Förderung des einem Patienten entnommenen Bluts durch den extrakorporalen Blutkreislauf 10 auf.

Die Dialysevorrichtung 1 weist weiterhin eine Steuervorrichtung 100 auf, die zur Steuerung der Behandlung eines Patienten eingerichtet ist. Die Steuervorrichtung 100 steuert beispielsweise die Funktion der Pumpe 9 oder schaltet Klemme 12.

Weiterhin ist eine Anzeigeeinrichtung 110 vorgesehen, mittels derer Betriebszustände der Dialysevorrichtung 1 angezeigt werden können. Die Anzeigeeinrichtung 110 ist in dem gezeigten Ausführungsbeispiel in Form eines schematisch dargestellten Monitors gezeigt. Es sind aber auch andere Anzeigeeinrichtungen, auch akustischer oder haptischer Art, denkbar.

Um Blut eines dialysepflichtigen Patienten mittels der Dialysevorrichtung zu behandeln, wird der Blutzulauf 13 mit dem Kreislauf des Patienten verbunden, beispielsweise über eine Kanüle. Über die Pumpe 9 wird das entnommene Blut zu dem Dialysefilter 5 gefördert, in welchem das Blut mittels bekannter Dialyseverfahren behandelt wird. In dem Dialysefilter 5 kann beispielsweise eine Hämodialyse, eine Hämofiltration, eine Hämodiafiltration oder ein anderes bekanntes Dialyseverfahren durchgeführt werden. Das behandelte Blut wird dann über den Blutrücklauf 14, welcher ebenfalls mit dem Kreislauf des Patienten verbunden ist, dem Patienten wieder zugeführt.

Über einen Differenzdrucksensor 6 wird ein Differenzdruck $p_{diffm}$ zwischen dem Druck $p_1$ im Blutzulauf 13, der einem arteriellen Druck entspricht, und dem Druck $p_2$ im Blutrücklauf 14, der einem venösen Druck entspricht, gemessen. Der gemessene Differenzdruck $p_{diffm}$ dient beispielsweise der Ermittlung eines Betriebszustandes der Dialysevorrichtung 1, beispielsweise zur Überwachung der korrekten Funktion der Dialysevorrichtung 1 bezüglich der Funktion weiterer Drucksensoren, die in der Figur 1 nicht gezeigt sind.

In der Figur 1 ist schematisch eine Ausbildung der Dialysevorrichtung 1 gezeigt, in der nur der Differenzdrucksensor 6 und eine Überwachungseinheit 11, die zur Ermittlung eines Betriebszustands auf Grundlage des von dem Differenzdrucksensor 6 gemessenen Differenzdrucks $p_{diffm}$ eingerichtet ist, vorgesehen sind.

Je nachdem, welcher Betriebszustand aufgrund des gemessenen Differenzdrucks $p_{diffm}$ ermittelt wurde, wird die Dialysebehandlung entweder in diesem Betriebszustand fortgesetzt, oder aber es erfolgt eine Fehlermeldung und/oder der Blutfluss im extrakorporalen Blutkreislauf wird gestoppt. Dazu kann der von der Überwachungseinheit 11 ermittelte Betriebszustand an die Steuervorrichtung 100 kommuniziert werden und die Steuervorrichtung 100 beendet entsprechend die Behandlung oder blockiert den Beginn einer Behandlung. Der ermittelte Betriebszustand kann auch alternativ oder zusätzlich an eine Anzeigeeinrichtung 110 kommuniziert werden und dort angezeigt werden.

Entspricht der gemessene Differenzdruck $p_{diffm}$ beispielsweise dem Sollwert eines normalen Dialysevorgangs, wird der Dialysevorgang fortgesetzt. Weicht der gemessene Differenzdruck $p_{diffm}$ jedoch von diesem Sollwert ab, beispielsweise in einem Fall, in dem die das behandelte Blut in den Patienten zurückführende venöse Nadel herausgerutscht ist oder eine andere Art von Nadeldiskonnektion stattgefunden hat, wodurch ein Druckabfall im Blutrücklauf 14 entsteht, wird dies von der Überwachungseinheit 11 bemerkt und es erfolgt eine Fehlermeldung an den Nutzer und/oder der extrakorporale Blutkreislauf wird sofort gestoppt, um eine Gefährdung des Patienten durch einen möglicherweise hohen Blutverlust zu verhindern.

Diese Alarmschwelle kann nicht nur bei der genannten Abweichung von einem Sollwert, sondern auch bei einer entsprechenden Veränderung des Differenzdrucks $p_{diffm}$ überschritten werden, so dass auch bei einer Veränderung des Differenzdrucks $p_{diffm}$ ein Alarm ausgelöst werden kann oder die Dialyse gestoppt werden kann. Beispielsweise kann bei einer abrupten Änderung des Differenzdrucks $p_{diffm}$ davon ausgegangen werden, dass eine Irregularität bezüglich der Integrität der Verbindung des extrakorporalen Blutkreislaufs mit dem Patienten vorliegt und mithin eine Nadeldiskonnektion vorliegen kann.

Die Auswertung des Verlaufs des Differenzdrucks $p_{diffm}$ über den Zeitverlauf hinweg beziehungsweise der Vergleich des gemessenen Differenzdrucks $p_{diffm}$ mit einem Sollwert erfolgt über die Überwachungseinheit 11, die zur Durchführung der entsprechenden Analysen eingerichtet ist. Die Überwachungseinheit 11 kann dabei beispielsweise in Form eines Hardwaremoduls und/oder in Form eines Softwaremoduls vorgesehen sein. Entsprechend des Ergebnisses der Auswertung sendet die Überwachungseinheit 11 dann eine Alarmnachricht und/oder einen Befehl zum Abbruch der Dialysebehandlung, sofern eine Alarmbedingung erreicht oder überschritten ist.

Ferner kann eine Steuereinrichtung 100 und/oder eine Anzeigeeinrichtung 110 der Dialysevorrichtung 1 vorgesehen sein. Die Steuereinrichtung 100 kann dazu eingerichtet sein, auf eine Nachricht der Überwachungseinheit 11 hin eine Dialysebehandlung abzubrechen und/oder eine zukünftige Behandlung zu blockieren. Die Anzeigeeinrichtung 110 kann dazu eingerichtet sein, eine Meldung auf der Grundlage des ermittelten Betriebszustandes auszugeben.

Die Überwachungseinheit 11 und/oder die Steuereinrichtung 100 und/oder die Anzeigeeinrichtung 110 können auch als eine Gesamteinrichtung, beispielsweise in Form eines Steuergeräts einer Dialysevorrichtung mit einem gemeinsamen Prozessor, ausgebildet sein.

Die Überwachungseinrichtung 11 kann beispielsweise so ausgebildet sein, dass sie das Signal des Differenzdrucksensors 6 empfängt und kann beispielsweise einen oder mehrere Prozessoren und einen Speicher zum Speichern eines Programms, mittels welchem die Überwachungsschritte auf dem Prozessor ausgeführt werden können, aufweisen. Die Überwachungseinrichtung 11 kann entsprechend als ein in der Dialysevorrichtung 1 integrierter Computer mit entsprechenden Datenleitungen realisiert sein.

Die Steuereinrichtung 100 kann auch darin bestehen, dass lediglich der Programmcode für die Steuerung sich von dem Programmcode der Überwachungseinrichtung 11 unterscheidet, jedoch zumindest teilweise dieselbe Hardware, beispielsweise ein gemeinsamer Prozessor, verwendet wird. Die Steuereinrichtung 100 und die Überwachungseinrichtung 11 können beispielsweise auch in unterschiedlichen Softwaremodulen vorliegen.

Die Steuereinrichtung 100 kann dazu programmiert sein, zum Blockieren oder Stoppen einer Behandlung mit der Dialysevorrichtung 1 ein Starten einer Blutpumpe zu verhindern oder diese zu stoppen. Das Blockieren kann auch darin bestehen, dass eine von einem Nutzer vorzunehmende Handlung nicht als ausführbar dargestellt wird. Beispielsweise kann ein Betätigungsfeld an der Maschine oder auf einem Bildschirm der Maschine nicht aktivierbar sein.

Die Anzeigeeinrichtung 110 kann in Form eines Bildschirms, beispielsweise in Form eines Berührungsbildschirms (Touch Screen) und/oder eines Lautsprechers zur Ausgabe akustischer Signale und/oder ein optischer Signalgeber wie beispielsweise eine Lampe vorliegen.

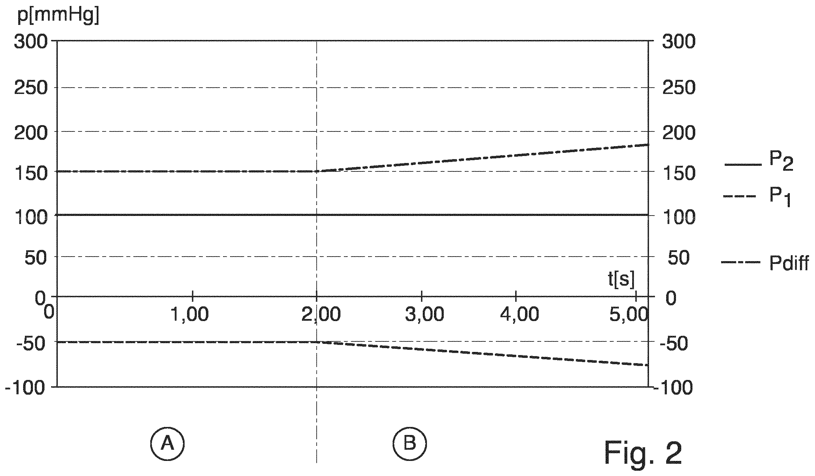

In Figur 2 ist ein Diagramm gezeigt, welches einen gewünschten Betriebszustand A und einen unerwünschten Betriebszustand B der Dialysevorrichtung 1 aus Figur 1 darstellt. Hierbei ist schematisch der Verlauf des Drucks p [mmHg] des gemessenen Differenzdrucks $p_{diffm}$ und schematisch der Verlauf des Drucks $p_1$ im Blutzulauf 13 und des Drucks $p_2$ im Blutrücklauf 14 über die Zeit t [s] aufgetragen. Die Werte des Drucks $p_1$ im Blutzulauf 13 und des Drucks $p_2$ im Blutrücklauf 14 sind hierbei zur Veranschaulichung nur schematisch dargestellt und werden mittels der Dialysevorrichtung 1 aus Figur 1 nicht notwendig direkt gemessen - in der Figur 1 sind entsprechend auch keine Druckmessvorrichtungen für die Drücke im Blutzulauf 13 und im Blutrücklauf 14 gezeigt.

Zur vereinfachten Darstellung sind die gezeigten Drücke linear dargestellt, was beispielsweise einem Mittelwert der Drücke entsprechen kann. Die tatsächlichen Drücke können natürlich mit dem Herzschlag und der Pumpbewegung der Pumpe 9 periodisch schwanken. Im gewünschten Betriebszustand A verlaufen der Druck $p_1$ im Blutzulauf 13 und der Druck $p_2$ im Blutrücklauf 14 im Wesentlichen zumindest bezüglich ihres Mittelwerts konstant, weshalb auch der mittels des Differenzdrucksensors 6 gemessene Differenzdruck $p_{diffm}$ im Wesentlichen konstant verläuft. In einem solchen Fall wird von der Überwachungseinheit 11 kein Fehleralarm und/oder Befehl zum Abbruch der Behandlung erzeugt.

Der unerwünschte Betriebszustand B zeigt schematisch den Fehlerfall, in dem die venöse Nadel, welche dem Patienten das gereinigte Blut zuführen soll, sich aus der Vene des Patienten gelöst hat (VND, Venous Needle Disconnect). Besteht keine Verbindung mehr zwischen der Nadel, also dem Ausgang des Blutrücklaufs 14, und der Vene des Patienten, kommt es zu einem (geringfügigen) Druckabfall des Drucks $p_2$ im Blutrücklauf 14. Aufgrund dieses Druckabfalls verändert sich auch der von dem Differenzdrucksensor gemessene Differenzdruck $p_{diffm}$. Die Überwachungseinheit 11 erfasst diese Veränderung des Druckabfalls des Differenzdrucks $p_{diffm}$ und sendet eine Fehlermeldung und/oder initiiert ein sofortiges Anhalten des Dialysevorgangs beziehungsweise der Pumpe 9.

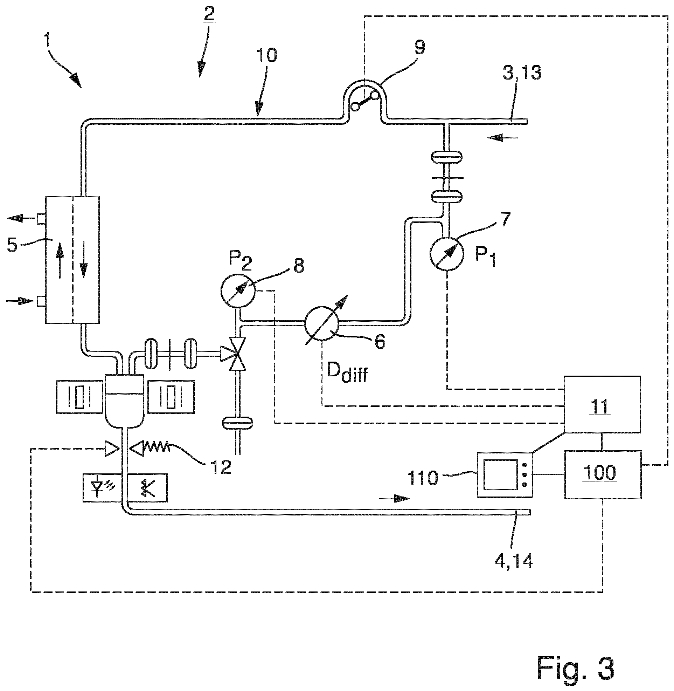

In Figur 3 ist schematisch eine Dialysevorrichtung 1 mit einem extrakorporalen Blutkreislauf 10 mit einem in einem ersten Abschnitt 3 angeordneten ersten Drucksensor 7, der hier in einem Blutzulauf 13 angeordnet ist, und einem in einem zweiten Abschnitt 4 angeordneten zweiten Drucksensor 8, der hier in einem Blutrücklauf 14 angeordnet ist, gezeigt. Ein Differenzdrucksensor 6 ist zwischen dem ersten Abschnitt 3 und dem zweiten Abschnitt 4 gezeigt. Zusätzlich weist die Dialysevorrichtung eine Überwachungseinheit 11 auf, die zum Ermitteln einer korrekten Funktion des ersten und/oder des zweiten Drucksensors 7, 8 eingerichtet ist und entsprechend auf diese Weise den Betriebszustand ermittelt.

Mittels der in Figur 3 gezeigten Dialysevorrichtung 1 ist es somit möglich, als Betriebszustand die Funktion des ersten und/oder des zweiten Drucksensors 7, 8 zu überprüfen.

Die Dialysevorrichtung 1 weist wieder eine Pumpe 9 zur Förderung des von einem Patienten entnommenen Blut und einen Dialysefilter 5 auf. Um Blut eines dialysepflichtigen Patienten mittels der Dialysevorrichtung 1 zu behandeln, wird der Blutzulauf 13 mit einer Vene des Patienten verbunden. Über die Pumpe 9 wird das dem Patienten entnommene Blut zu dem Dialysefilter 5 gefördert, in welchem mittels eines bekannten Dialyseverfahrens, beispielsweise Hämodialyse, Hämofiltration, Hämodiafiltration oder eines anderen Dialyseverfahrens, das Blut des Patienten gereinigt wird.

Das gereinigte Blut wird dann über den Blutrücklauf 14, welche ebenfalls mit dieser Vene des Patienten verbunden ist, dem Patienten zugeführt. Über den Differenzdrucksensor 6 wird der Differenzdruck $p_{diffm}$ zwischen dem Druck $p_1$ im Blutzulauf 13, also dem ersten Abschnitt 3 des Flüssigkeitsleitungssystems 2, und dem Druck $p_2$ im

Blutrücklauf 14, also dem zweiten Abschnitt 4 des Flüssigkeitsleitungssystems 2 gemessen. Zusätzlich wird über den ersten Drucksensor 7 der Druck $p_1$ im ersten Abschnitt 3 und über einen zweiten Drucksensor 8 der Druck $p_2$ im zweiten Abschnitt 4 gemessen.

Wird der gemessene Differenzdruck $p_{diffm}$ isoliert betrachtet, kann - wie bereits zu Figur 1 beschrieben - eine Aussage über einen Betriebszustand der Dialysevorrichtung 1 gemacht werden.

Um nun eine Überwachung des ersten und/oder des zweiten Drucksensors 7, 8 vorzunehmen, wird in der Überwachungseinheit 11 über den gemessenen Druck $p_1$ im ersten Abschnitt 3 und den gemessenen Druck $p_2$ im zweiten Abschnitt 4 ein Differenzdruck $p_{diffb}$ berechnet. Dann werden der mittels des Differenzdrucksensors 6 gemessene Differenzdruck $p_{diffm}$ und der berechnete Differenzdruck $p_{diffb}$ miteinander verglichen, beispielsweise voneinander subtrahiert. Aus dem Vergleich zwischen dem gemessenen Differenzdruck $p_{diffm}$ und dem berechnete Differenzdruck $p_{diffb}$ ergibt sich ein resultierender Druck $p_{res}$.

Für den Fall, dass beide Drucksensoren 7, 8 korrekt funktionieren, d.h., dass der Drucksensor 7 den Druck $p_1$ im ersten Abschnitt 3 und der Drucksensor 8 den Druck $p_2$ im zweiten Abschnitt 4 korrekt misst, stimmen der gemessene Differenzdruck und der berechnete Differenzdruck überein, d.h. der resultierende Druck $p_{res}$ ist Null oder zumindest über den Zeitverlauf hinweg konstant.

Für den Fall, dass beide oder einer der beiden Drucksensoren 7, 8 nicht funktionieren, d.h., dass der Drucksensor 7 den Druck $p_1$ im ersten Abschnitt 3 und/oder der Drucksensor 8 den Druck $p_2$ im zweiten Abschnitt 4 nicht korrekt misst, stimmen der gemessene Differenzdruck $p_{diffm}$ und der berechnete Differenzdruck $p_{diffb}$ nicht überein, d.h. der resultierende Druck $p_{res}$ ist ungleich Null oder über den Zeitverlauf hinweg veränderlich. Die Auswertung des resultierende Drucks $p_{res}$, d.h. die Berechnung des resultierende Druck $p_{res}$ aus dem gemessenen Differenzdruck $p_{diffm}$ und dem berechneten Differenzdruck $p_{diffb}$ und den Vergleich mit einem Sollwert (beispielsweise Null) erfolgt über die Überwachungseinheit 11. Wird eine Abweichung des resultierenden Drucks $p_{res}$ von dem Sollwert detektiert, erfolgt eine Fehlermeldung und/oder der extrakorporale Blutkreislauf 10 wird gestoppt.

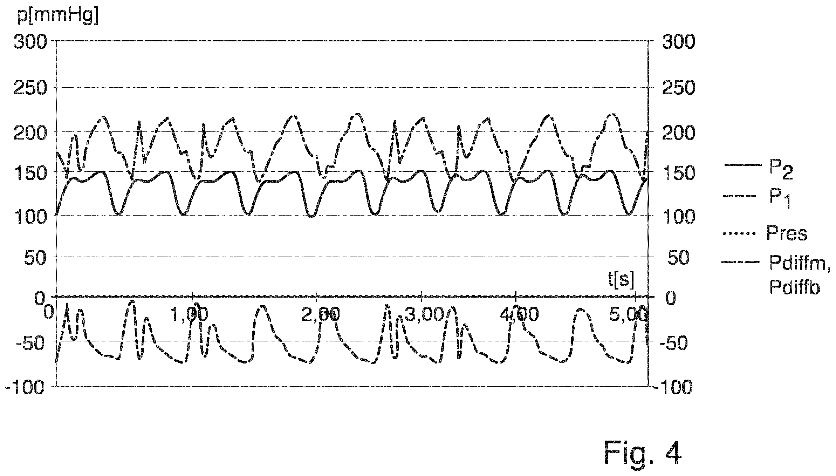

Ein beispielhafter Druckverlauf für den Fall, dass beide Drucksensoren 7, 8 korrekt messen, ist in dem schematischen Diagramm in Figur 4 gezeigt. Das Diagramm in Figur 4 zeigt dabei die gemessenen Drücke $p_1$, $p_2$, $p_{diffm}$ und die berechneten Drücke $p_{diffb}$ und $p_{res}$. Dabei ist jeweils der Druckverlauf p [mmHg] über die Zeit t [s] aufgetragen. Wie aus dem Diagramm ersichtlich ist, stimmen der berechnete Differenzdruck $p_{diffb}$ und der gemessene Differenzdruck $p_{diffm}$ überein, d.h. die Differenz zwischen dem berechneten Differenzdruck $p_{diffb}$ und der gemessenen Differenzdruck $p_{diffm}$ ist Null. In diesem Fall erfolgt keine Fehlermeldung der Überwachungseinheit 11 und das Dialyseverfahren wird fortgesetzt.

In einem Falle, in dem toleranzbehaftete Drucksensoren 6, 7, 8 verwendet werden, wird zwischen dem gemessenen und damit toleranzbehafteten Differenzdruck $p_{diffm}$ und dem ebenfalls toleranzbehafteten, da aus zwei toleranzbehafteten Messwerten $p_1$, $p_2$ berechneten Differenzdruck $p_{diffb}$ ein Unterschied bestehen, so dass der resultierende Druck nicht gleich Null ist, aber im Wesentlichen im Zeitverlauf konstant ist. Die Analyse durch die Überwachungseinheit 11 kann dem Rechnung tragen und eine Abweichung des resultierenden Drucks von der Nulllinie im Bereich einer vorgegebenen Toleranz immer noch als keinen Fehlerfall ansehen.

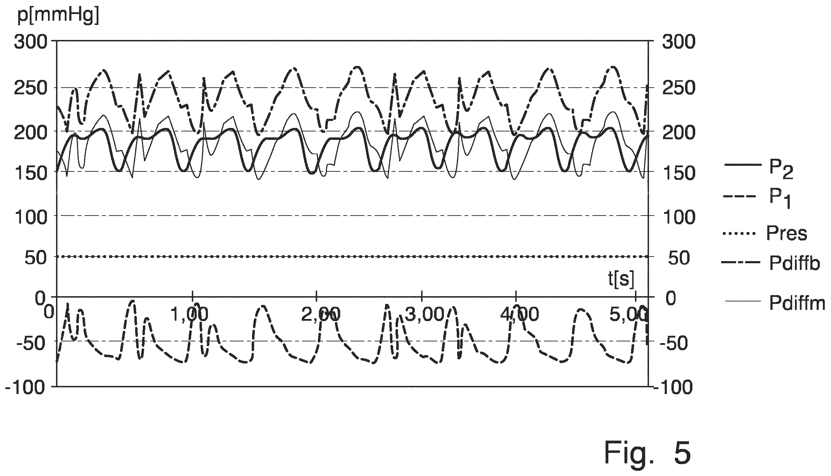

In Figur 5 ist ein schematisches Diagramm dargestellt, welches einen beispielhaften Druckverlauf für den Fall zeigt, dass der zweite Drucksensor 8, welcher den Druck $p_2$ im Blutrücklauf messen soll, nicht korrekt misst. Das Diagramm in Figur 5 zeigt dabei die gemessenen Drücke $p_1$, $p_2$, $p_{diffm}$ und die berechneten Drücke $p_{diffb}$ und $p_{res}$. Dabei ist der Druckverlauf p [mmHg] über die Zeit t [s] aufgetragen.

Wie aus dem Diagramm ersichtlich ist, stimmen der berechnete Differenzdruck $p_{diffb}$ und der gemessene Differenzdruck $p_{diffm}$ nicht überein, d.h. die Differenz zwischen dem berechneten Differenzdruck $p_{diffb}$ und der gemessenen Differenzdruck $p_{diffm}$ ist ungleich Null und die Abweichung überschreitet eine vorgegebene Toleranz. Die Überwachungseinheit 11 detektiert diese Abweichung des Differenzdrucks $p_{diffm}$ von dem Sollwert und sendet eine Fehlermeldung oder stoppt den extrakorporalen Blutkreislauf 10.

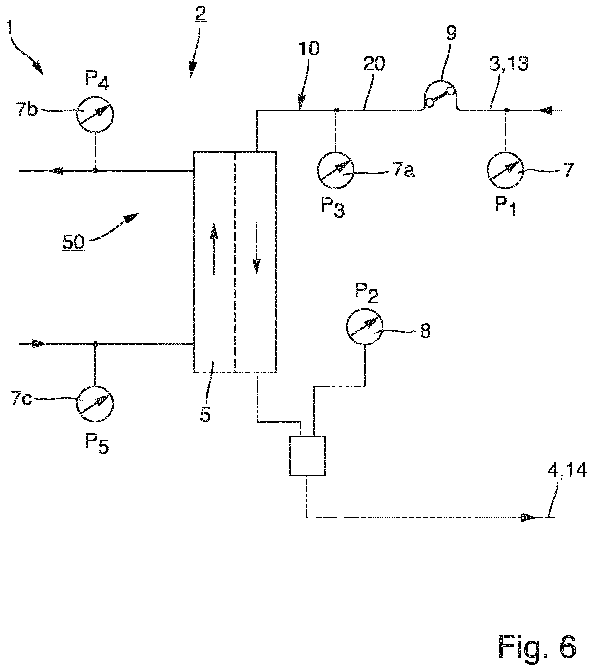

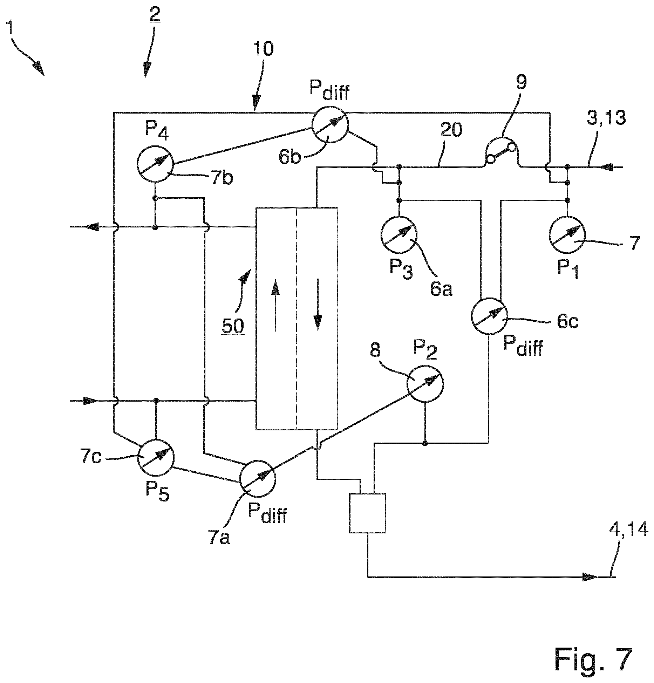

In den Figuren 6 und 7 ist jeweils schematisch eine Dialysevorrichtung 1 mit einem extrakorporalen Blutkreislauf 10 mit einem ersten Drucksensor 7 in einem ersten Abschnitt 3, der dem Blutzulauf 13 entspricht, und mit einem zweiten Drucksensor 8 in einem zweiten Abschnitt 4, der dem Blutrücklauf 14 entspricht, gezeigt. Wie oben ausgeführt, können Differenzdrucksensoren zwischen verschiedenen Abschnitten des Flüssigkeitsleitungssystems 2 angeordnet sein und die Funktion der an diesen Abschnitten angeordneten Drucksensoren kann entsprechend mittels des jeweiligen Differenzdrucksensors überwacht werden.

In Figur 6 sind entsprechend exemplarisch verschiedene Abschnitte des Flüssigkeitsleitungssystems 2 sowie den Druck in diesen Abschnitten messende Drucksensoren gezeigt.

So sind Drucksensoren beispielsweise in Form eines Drucksensors 7 zum Bestimmen eines Drucks $p_1$ zwischen einem arteriellen Ende des arteriellen Blutzulaufs 13 und der Blutpumpe 9, eines Drucksensors 8 zum Bestimmen eines Drucks $p_2$ zwischen einem venösen Ende des venösen Blutrücklaufs 13 und dem Dialysator oder Dialysefilter 5, eines Drucksensors 7a zum Bestimmen eines Drucks $p_3$ in einem Abschnitt 20 des Flüssigkeitsleitungssystems 2 zwischen der Blutpumpe 9 und dem Dialysator 5, eines Drucksensors 7b zum Bestimmen eines Drucks $p_4$ in einer Dialysatleitung stromabwärts des Dialysators 5, und/oder eines Drucksensors 7c zum Bestimmen eines Drucks $p_5$ in der Dialysatleitung

stromaufwärts des Dialysators 5 gezeigt. Der Dialysator 5 bildet mit seinen Zu- und Ableitungen eine Dialysierflüssigkeitskreislauf 50 aus.

In Figur 7 sind verschiedene Abschnitte des Flüssigkeitsleitungssystems 2 und entsprechende Differenzdrucksensoren zum Messen des Differenzdrucks zwischen diesen Abschnitten gezeigt.

Hierbei sind in dieser Figur verschiedene Anordnungsmöglichkeiten von Differenzdrucksensoren 6a bis 6c veranschaulicht. Dabei können die Differenzdrucksensoren 6a bis 6c in Abschnitten von Bereichen verschiedener Druckregime liegen. Die Dialysevorrichtung 1, welche in den Figuren 6 und 7 gezeigt ist, weist entsprechend der Drucksensoren 7a bis 7c und der Differenzdrucksensoren 6a bis 6c jeweils eigene oder kombinierte Überwachungseinheiten auf, die jedoch zur besseren Übersicht nicht dargestellt sind.

In den Abschnitten, zwischen denen ein Differenzdrucksensor 6a bis 6c angeordnet ist, kann ein Element liegen, das durch seinen Widerstand die Druckübertragung von einem ersten Abschnitt 3, beispielsweise einem Blutzulauf 13, in einen zweiten Abschnitt 4, beispielsweise einem Blutrücklauf 14, beeinflusst. Dieses Element kann beispielsweise eine Pumpe, insbesondere eine peristaltische Pumpe, Hohlfasern einer Dialysemembran, eine Dialysemembran, ein Flussminderer, ein Ventil, eine bei Betrieb teilweise gefüllte Kammer oder ein vergleichbares Element sein. Es kann sich bei diesem Element auch um ein druckerzeugendes Element handeln, beispielsweise um eine Pumpe, wobei die Druckschwankungen vor und hinter diesem Element in Phase zueinander stehen.

Handelt es sich bei diesen druckändernden Elementen um passive Elemente und werden die Drücke in den beiden Abschnitten 3, 4 von völlig verschiedenen Druckerzeugern, beispielsweise von zwei unabhängig voneinander agierende Pumpen, hervorgerufen, kann es erforderlich sein, über einen vorgegebenen Zeitraum hinweg das Signal zu mitteln, um diese unabhängigen Schwankungen der Drucksignale zu einem stabilen Wert zu mitteln. Dies kann beispielsweise auch dann der Fall sein, wenn der erste Abschnitt im extrakorporalen Blutkreislauf 10 und der zweite Abschnitt auf der Seite des Dialysierflüssigkeitskreislaufs 50 liegt.

Die Dialysevorrichtung 1 kann auch einen Differenzdrucksensor 6a bis 6c aufweisen, der mit mehr als zwei Abschnitten 3 ,4 verbunden ist. Weiterhin kann eine Steuerung (nicht gezeigt) vorgesehen sein, die dazu konfiguriert ist, jeweils genau zwei Abschnitte 3, 4 über den Differenzdrucksensor 6a bis 6c zu verbinden und für diese Abschnitte 3, 4 und/oder die mit diesen Abschnitten verbundenen Drucksensoren 7a bis 7c eines der beschriebenen Überwachungsverfahren durchzuführen.

In einer Speichereinheit der Dialysevorrichtung 1 kann gespeichert sein, wie lange oder über wie viele Messwerte hinweg die Mittelung der Drucksignale erfolgen muss, in Abhängigkeit der jeweils verbundenen Abschnitte oder Drucksensoren.

Ein weiterer, in der Speichereinheit abgelegter Parameter für die Dauer oder die Anzahl der Messwerte für die Mittelung kann dabei auch eine Geschwindigkeit sein, mit der die Pumpen der Dialysevorrichtung die Flüssigkeit durch die jeweiligen Abschnitte 3, 4 transportieren. Dazu kann beispielsweise eine Tabelle oder eine Gleichung in der Speichereinheit hinterlegt sein. Für den Fall, dass die Drücke in den einzelnen Abschnitten 3, 4 durch jeweils unabhängige Drucksensoren gemessen werden, können mittels eines in einem Prozessor der Dialysevorrichtung 1 ablaufenden Programms die Druckverläufe solange gemittelt werden, bis die Schwankungen der Druckverläufe unter einen im Speicher abgelegten, vorgegebenen Grenzwert gefallen sind.

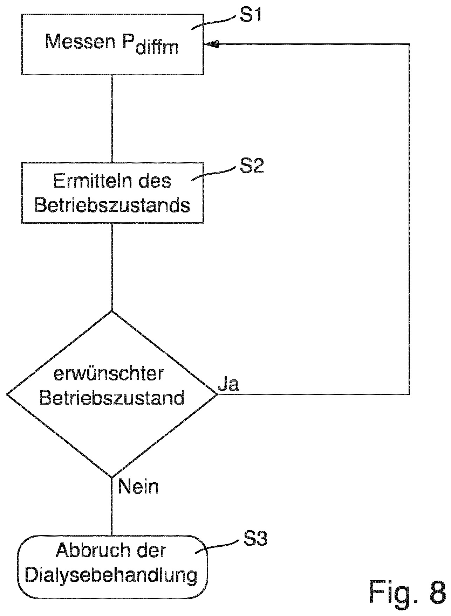

In Figur 8 ist eine schematische Darstellung eines Verfahrens zur Überwachung eines Betriebszustands einer Dialysevorrichtung gezeigt. Das Verfahren wird bevorzugt mit einer zuvor beschriebenen Dialysevorrichtung 1 durchgeführt, welche ein Flüssigkeitsleitsystem 2 mit einem ersten Abschnitt 3 und einem zweiten Abschnitt 4 umfasst.

In einem ersten Schritt S1 des dargestellten Verfahrens wird ein Differenzdruck $p_{diffm}$ zwischen einem ersten Druck $p_1$ in dem Abschnitt 3 und einem zweiten Druck $p_2$ in dem zweiten Abschnitt 4 mittels eines Differenzdrucksensors 6 gemessen.

In einem zweiten Schritt S2 wird auf Grundlage des gemessenen Differenzdrucks $p_{diffm}$ ein Betriebszustand ermittelt.

Wird ein unerwünschter Betriebszustand in Schritt S2 ermittelt, beispielsweise in dem Fall, in dem die venöse Nadel, über welche das gereinigte Blut dem Patienten zugeführt wird, versehentlich von dem venösen Patientenzugang des Patienten gelöst wird (VND, Venous Needle Disconnect), wird eine Meldung, beispielsweise über eine Anzeigeeinrichtung 110, ausgegeben. Mittels dieser Meldung wird dem Nutzer signalisiert, dass ein unerwünschter Betriebszustand vorliegt, wobei der Nutzer dann sofort entsprechende Maßnahmen einleiten kann.

Ergibt die Ermittlung des Betriebszustandes in Schritt S2, dass ein erwünschter Betriebszustand vorliegt, beispielsweise in dem Fall, in dem der Differenzdruck annähernd konstant ist und die Dialyse somit effektiv und sicher durchgeführt werden kann, wird die Behandlung fortgesetzt.

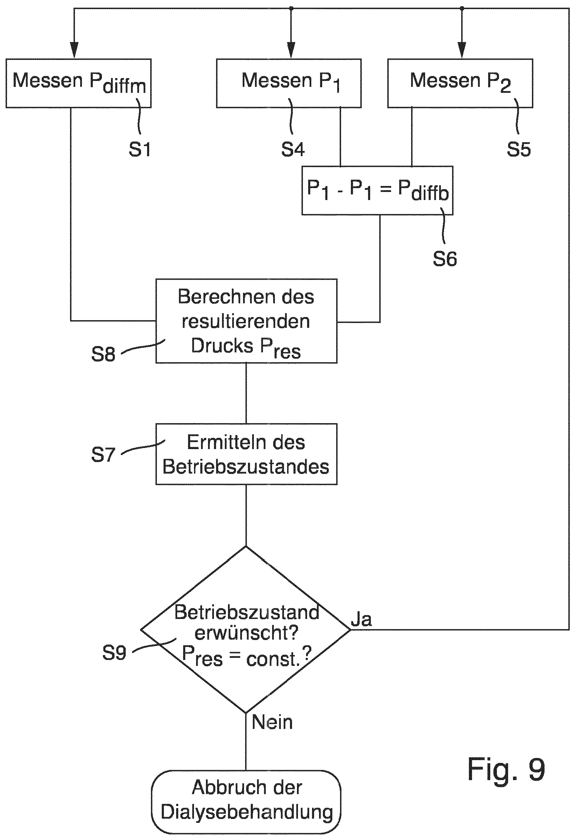

In Figur 9 ist eine schematische Darstellung eines Verfahrens zur Überwachung eines Betriebszustands einer Dialysevorrichtung 1 auf Grundlage eines berechneten Differenzdrucks $p_{diffb}$ und eines gemessenen Differenzdrucks $p_{diffm}$ gezeigt.

In einem Schritt S4 wird ein erster Druck $p_1$ in einem ersten Abschnitt 3 gemessen. In einem Schritt S5 wird ein zweiter Druck $p_2$ in einem zweiten Abschnitt 4 gemessen. Mittels des gemessenen ersten Drucks $p_1$ und des gemessenen

zweiten Drucks $p_2$ wird in einem Schritt S6 der Differenzdrucks $p_{diffb}$ berechnet.

Parallel dazu, danach oder auch davor wird in einem Schritt S1 ein Differenzdruck $p_{diffm}$ zwischen dem ersten Druck $p_1$ in dem Abschnitt 3 und dem zweiten Druck $p_2$ in dem zweiten Abschnitt 4 mittels eines Differenzdrucksensors 6 gemessen.

Mittels des berechneten Differenzdrucks $p_{diffb}$ und des gemessenen Differenzdrucks $p_{diffm}$ wird in einem Schritt S8 ein resultierender Druck $p_{res}$ berechnet.

Auf der Grundlage des berechneten ein resultierenden Drucks $p_{res}$ wird in einem Schritt S7 der Betriebszustand der Dialysevorrichtung 1 ermittelt und einem Schritt S9 eine Analyse durchgeführt. Soweit anwendbar, können alle einzelnen Merkmale, die in den Ausführungsbeispielen dargestellt sind, miteinander kombiniert und/oder ausgetauscht werden, ohne den Bereich der Erfindung zu verlassen, wie sie in den Ansprüchen definiert ist.

Bezugszeichenliste

| | |
|---|---|
| 1 | Dialysevorrichtung |
| 2 | Flüssigkeitsleitungssystem |
| 3 | erster Abschnitt |
| 4 | zweiter Abschnitt |
| 5 | Dialysefilter |
| 6 | Differenzdrucksensor |
| 6a - 6c | Differenzdrucksensor |
| 7 | erster Drucksensor |
| 7a - 7c | Drucksensor |
| 8 | zweiter Drucksensor |
| 9 | Pumpe |
| 10 | extrakorporaler Blutkreislauf |
| 11 | Überwachungseinheit zur Ermittlung eines Betriebszustandes |
| 12 | Klemme |
| 13 | Blutzulauf |
| 14 | Blutrücklauf |
| 100 | Steuervorrichtung |
| 110 | Anzeigeeinrichtung |
| $p_1$ | erster Druck |
| $p_2$ | zweiter Druck |
| $p_{art}$ | arterieller Druck |
| $p_{ven}$ | venöser Druck |
| $p_{diffm}$ | gemessener Differenzdruck |
| $p_{diffb}$ | berechneter Differenzdruck |
| $p_{res}$ | resultierender Differenzdruck |
| A | gewünschter Betriebszustand |
| B | unerwünschter Betriebszustand |
| S1 | Messen eines Differenzdrucks $p_{diffm}$ |
| S2 | Ermitteln des Betriebszustandes |
| S3 | Abbrechen einer Dialysebehandlung |
| S4 | Messen des Drucks $p_1$ |
| S5 | Messen des Drucks $p_2$ |
| S6 | Berechnen des Differenzdrucks $p_{diffb}$ |
| S7 | Ermitteln des Betriebszustands |
| S8 | Berechnen eines resultierenden Drucks $p_{res}$ |
| S9 | Analyse |

**Patentansprüche**

1. Dialysevorrichtung (1) zur Durchführung einer Dialysebehandlung, umfassend ein Flüssigkeitsleitungssystem (2), welches einen ersten Abschnitt (3) und einen zweiten Abschnitt (4) umfasst,

   wobei ein Differenzdrucksensor (6) zur Messung eines Differenzdrucks $p_{diffm}$ zwischen einem ersten Druck ($p_1$)

in dem ersten Abschnitt (3) des Flüssigkeitsleitungssystems (2) und einem zweiten Druck ($p_2$) in dem zweiten Abschnitt (4) des Flüssigkeitsleitungssystems (2) vorgesehen ist, wobei eine Überwachungseinheit (11) vorgesehen ist, die zur Ermittlung eines Betriebszustandes auf Grundlage des gemessenen Differenzdrucks $p_{diffm}$ eingerichtet ist, und wobei eine Steuervorrichtung (100) vorgesehen ist, die dazu eingerichtet ist, die Dialysebehandlung entsprechend des ermittelten Betriebszustandes abzubrechen und/oder zu blockieren, und/oder dass eine Anzeigeeinrichtung (110) vorgesehen ist, die zum Ausgeben einer Meldung auf der Grundlage des ermittelten Betriebszustandes eingerichtet ist, **dadurch gekennzeichnet, dass** ein erster Drucksensor (7) zur Messung eines Drucks ($p_1$) im ersten Abschnitt (3) und ein zweiter Drucksensor (8) zur Messung eines Drucks ($p_2$) im zweiten Abschnitt (4) vorgesehen ist, und dass die Überwachungseinheit (11) dazu eingerichtet ist, aus dem ersten gemessenen Druck ($p_1$) im ersten Abschnitt (3) und dem zweiten gemessenen Druck ($p_2$) im zweiten Abschnitt (4) einen Differenzdruck $p_{diffb}$ zu berechnen und wobei die Überwachungseinheit (11) dazu eingerichtet ist, auf Grundlage des berechneten Differenzdrucks $p_{diffb}$ und des gemessenen Differenzdrucks $p_{diffm}$ den Betriebszustand zu bestimmen.

2. Dialysevorrichtung (1) gemäß Anspruch 1, **dadurch gekennzeichnet, dass** der Betriebszustand eine inkorrekte Funktion des ersten und/oder des zweiten Drucksensors (6, 7, 8) umfasst.

3. Dialysevorrichtung (1) gemäß Anspruch 1 oder 2, **dadurch gekennzeichnet, dass** zur Bestimmung des Betriebszustands die Überwachungseinheit (11) dazu eingerichtet ist, eine Differenz $p_{res}$ zwischen dem berechneten Differenzdruck $p_{diffb}$ und dem gemessenen Differenzdruck $p_{diffm}$ zu bestimmen und die berechnete Differenz $p_{res}$ mit einem Sollwert zu vergleichen.

4. Dialysevorrichtung (1) gemäß einem der Ansprüche 1 bis 3, **dadurch gekennzeichnet, dass** der erste Abschnitt (3) und der zweite Abschnitt (4) des Flüssigkeitsleitungssystems (2) Teile eines extrakorporalen Blutkreislaufs (10) mit einem Dialysefilter (5) aufweist, wobei der erste Abschnitt (3) einem Blutzulauf (13) und der zweite Abschnitt (4) einem Blutrücklauf (14) entspricht.

5. Dialysevorrichtung (1) gemäß Anspruch 4, **dadurch gekennzeichnet, dass** die Überwachungseinheit (11) dazu eingerichtet ist, auf Grundlage des gemessenen Differenzdrucks $p_{diffm}$ und/oder einer Veränderung des gemessenen Differenzdrucks $p_{diffm}$ eine Nadeldiskonnektion als Betriebszustand zu detektieren.

6. Verfahren zur Überwachung eines Betriebszustands einer Dialysevorrichtung (1), die ein Flüssigkeitsleitungssystem (2) gefüllt mit einer anderen Flüssigkeit als Blut mit einem ersten Abschnitt (3) und einem zweiten Abschnitt (4) umfasst, umfassend die Schritte

  - Messen (S1) eines Differenzdrucks $p_{diffm}$ zwischen einem ersten Druck ($p_1$) im ersten Abschnitt (3) und einem zweiten Druck ($p_2$) im zweiten Abschnitt (4) mittels eines Differenzdrucksensors (6),
  - Ermitteln (S2) des Betriebszustandes auf Grundlage des gemessenen Differenzdrucks $p_{diffm}$, und
  - Ausgeben einer Meldung auf Grundlage des ermittelten Betriebszustandes,
  **dadurch gekennzeichnet, dass** das Verfahren die Schritte umfasst
  - Messen (S4) eines Drucks ($p_1$) im ersten Abschnitt (3) mittels eines ersten Drucksensors (7),
  - Messen (S5) eines Drucks ($p_2$) im zweiten Abschnitt (4) mittels eines zweiten Drucksensors (8),
  - Berechnen (S6) eines Differenzdrucks $p_{diffb}$ zwischen dem gemessenen Druck ($p_1$) im ersten Abschnitt (3) und dem gemessenen Druck ($p_2$) im zweiten Abschnitt (4),
  - Ermitteln (S7) des Betriebszustands auf der Grundlage des berechneten Differenzdrucks $p_{diffb}$ und des gemessenen Differenzdrucks $p_{diffm}$,

  wobei das Verfahren vor und/oder nach einer Behandlung durchgeführt wird.

7. Verfahren gemäß Anspruch 6, **dadurch gekennzeichnet, dass** der Betriebszustand eine inkorrekte Funktion des ersten und/oder des zweiten Drucksensors (7, 8) umfasst.

8. Verfahren gemäß einem der Ansprüche 6 oder 7, **dadurch gekennzeichnet, dass** das Ermitteln des Betriebszustandes folgende Schritte umfasst:

  - Berechnen (S8) eines resultierenden Drucks $p_{res}$ aus der Differenz zwischen dem berechneten Differenzdruck $p_{diffb}$ und dem gemessenen Differenzdruck $p_{diffm}$, und

- Analyse (S9) des Verhaltens des Drucks $p_{res}$

9. Verfahren gemäß Anspruch 8, **dadurch gekennzeichnet, dass** die Analyse (S9) ein Abgleich mit einem Sollwert und/oder eine Analyse der Veränderung über die Zeit ist.

10. Verfahren gemäß Anspruch 9, **dadurch gekennzeichnet, dass** bei einer Abweichung des resultierenden Drucks $p_{res}$ vom Sollwert aus dem resultierenden Druck $p_{res}$ ein Fehlervolumen kumuliert wird und bei Überschreiten eines vorgegebenen maximalen Fehlervolumens die Meldung erfolgt.

11. Verfahren gemäß einem der Ansprüche 9 oder 10, **dadurch gekennzeichnet, dass** bei einer anhaltenden Abweichung des resultierenden Drucks $p_{res}$ vom Sollwert bei Erreichen einer Auslöseschwelle die Meldung erfolgt.

12. Verfahren gemäß einem der Ansprüche 7 bis 11, **dadurch gekennzeichnet, dass** nach Ermittlung einer inkorrekten Funktion eines ersten Drucksensors (7) in einem ersten Abschnitt (3) aus dem gemessenen Differenzdruck $p_{diffm}$ und dem gemessenen Druck ($p_2$) in einem zweiten Abschnitt (4) der Druck (p1) im ersten Abschnitt (3) bestimmt wird.


## Claims

1. Dialysis device (1) for carrying out a dialysis treatment, comprising a liquid line system (2) which comprises a first section (3) and a second section (4),

   wherein a differential pressure sensor (6) is provided for measuring a differential pressure $p_{diffm}$ between a first pressure ($p_1$) in the first section (3) of the liquid line system (2) and a second pressure ($p_2$) in the second section (4) of the liquid line system (2),
   wherein a monitoring unit (11) is provided which is configured to determine an operating state on the basis of the measured differential pressure $p_{diffm}$, and
   wherein a control device (100) is provided which is configured to abort and/or to block the dialysis treatment in accordance with the determined operating state, and/or in that a display device (110) is provided which is configured to output a message on the basis of the determined operating state,
   **characterized in that**
   a first pressure sensor (7) is provided for measuring a pressure ($p_1$) in the first section (3) and a second pressure sensor (8) is provided for measuring a pressure ($p_2$) in the second section (4), and **in that** the monitoring unit (11) is configured to calculate a differential pressure $p_{diffb}$ from the first measured pressure ($p_1$) in the first section (3) and the second measured pressure ($p_2$) in the second section (4), and wherein the monitoring unit (11) is configured to determine the operating state on the basis of the calculated differential pressure $p_{diffb}$ and the measured differential pressure $p_{diffm}$.

2. Dialysis device (1) according to claim 1, **characterized in that** the operating state comprises an incorrect function of the first and/or of the second pressure sensor (6, 7, 8).

3. Dialysis device (1) according to claim 1 or 2, **characterized in that,** in order to determine the operating state, the monitoring unit (11) is configured to determine a difference $p_{res}$ between the calculated differential pressure $p_{diffb}$ and the measured differential pressure $p_{diffm}$ and to compare the calculated difference $p_{res}$ with a setpoint value.

4. Dialysis device (1) according to one of claims 1 to 3, **characterized in that** the first section (3) and the second section (4) of the liquid line system (2) have parts of an extracorporeal blood circuit (10) with a dialysis filter (5), wherein the first section (3) corresponds to a blood inflow (13) and the second section (4) corresponds to a blood return flow (14).

5. Dialysis device (1) according to claim 4, **characterized in that** the monitoring unit (11) is configured to detect a needle disconnection as an operating state on the basis of the measured differential pressure $p_{diffm}$ and/or a change in the measured differential pressure pdiffm.

6. Method for monitoring an operating state of a dialysis device (1) which comprises a liquid line system (2) filled with a liquid other than blood with a first section (3) and a second section (4), comprising the steps of

   - measuring (S1) a differential pressure $p_{diffm}$ between a first pressure ($p_1$) in the first section (3) and a second pressure ($p_2$) in the second section (4) by means of a differential pressure sensor (6),

- determining (S2) the operating state on the basis of the measured differential pressure $p_{diffm}$, and
- outputting a message on the basis of the determined operating state,
**characterized in that** the method comprises the steps of
- measuring (S4) a pressure ($p_1$) in the first section (3) by means of a first pressure sensor (7),
- measuring (S5) a pressure ($p_2$) in the second section (4) by means of a second pressure sensor (8),
- calculating (S6) a differential pressure $p_{diffb}$ between the measured pressure ($p_1$) in the first section (3) and the measured pressure ($p_2$) in the second section (4),
- determining (S7) the operating state on the basis of the calculated differential pressure $p_{diffb}$ and the measured differential pressure $p_{diffm}$,

wherein the method is carried out before and/or after a treatment.

7. Method according to claim 6, **characterized in that** the operating state comprises an incorrect function of the first and/or of the second pressure sensor (7, 8).

8. Method according to one of claims 6 or 7, **characterized in that** the determination of the operating state comprises the following steps:

   - calculating (S8) a resulting pressure $p_{res}$ from the difference between the calculated differential pressure $p_{diffb}$ and the measured differential pressure $p_{diffm}$, and
   - analyzing (S9) the behavior of the pressure $p_{res}$.

9. Method according to claim 8, **characterized in that** the analysis (S9) is a comparison with a setpoint value and/or an analysis of the change over time.

10. Method according to claim 9, **characterized in that,** in the event of a deviation of the resulting pressure $p_{res}$ from the setpoint value, an error volume is accumulated from the resulting pressure $p_{res}$ and the message is issued when a predefined maximum error volume is exceeded.

11. Method according to one of claims 9 or 10, **characterized in that,** in the event of a sustained deviation of the resulting pressure $p_{res}$ from the setpoint value, the message is issued when a trigger threshold is reached.

12. Method according to one of claims 7 to 11, **characterized in that,** after determination of an incorrect function of a first pressure sensor (7) in a first section (3), the pressure (p1) in the first section (3) is determined from the measured differential pressure $p_{diffm}$ and the measured pressure ($p_2$) in a second section (4).


**Revendications**

1. Dispositif de dialyse (1) pour effectuer un traitement de dialyse, comprenant un système de conduite de liquide (2) qui comprend une première section (3) et une deuxième section (4),

   dans lequel un capteur de pression différentielle (6) est prévu pour mesurer une pression différentielle $p_{diffm}$ entre une première pression ($p_1$) dans la première section (3) du système de conduite de liquide (2) et une deuxième pression ($p_2$) dans la deuxième section (4) du système de conduite de liquide (2),
   dans lequel une unité de surveillance (11) est prévue, laquelle est conçue pour déterminer un état de fonctionnement sur la base de la pression différentielle $p_{diffm}$ mesurée, et
   dans lequel un dispositif de commande (100) est prévu, lequel est conçu pour interrompre et/ou bloquer le traitement de dialyse en fonction de l'état de fonctionnement déterminé, et/ou en ce qu'un dispositif d'affichage (110) est prévu, lequel est conçu pour émettre un message sur la base de l'état de fonctionnement déterminé, **caractérisé en ce que**
   un premier capteur de pression (7) est prévu pour mesurer une pression ($p_1$) dans la première section (3) et un deuxième capteur de pression (8) est prévu pour mesurer une pression ($p_2$) dans la deuxième section (4), et **en ce que** l'unité de surveillance (11) est conçue pour calculer une pression différentielle $p_{diffb}$ à partir de la première pression mesurée ($p_1$) dans la première section (3) et de la deuxième pression mesurée ($p_2$) dans la deuxième section (4) et dans lequel l'unité de surveillance (11) est conçue pour déterminer l'état de fonctionnement sur la base de la pression différentielle $p_{diffb}$ calculée et de la pression différentielle $p_{diffm}$ mesurée.

**2.** Dispositif de dialyse (1) selon la revendication 1, **caractérisé en ce que** l'état de fonctionnement comprend une fonction incorrecte du premier et/ou du deuxième capteur de pression (6, 7, 8).

**3.** Dispositif de dialyse (1) selon la revendication 1 ou 2, **caractérisé en ce que** pour déterminer l'état de fonctionnement, l'unité de surveillance (11) est conçue pour déterminer une différence $p_{res}$ entre la pression différentielle $p_{diffb}$ calculée et la pression différentielle $p_{diffm}$ mesurée et pour comparer la différence $p_{res}$ calculée à une valeur de consigne.

**4.** Dispositif de dialyse (1) selon l'une quelconque des revendications 1 à 3, **caractérisé en ce que** la première section (3) et la deuxième section (4) du système de conduite de liquide (2) présentent des parties d'un circuit sanguin extracorporel (10) avec un filtre de dialyse (5), dans lequel la première section (3) correspond à une arrivée de sang (13) et la deuxième section (4) correspond à un retour de sang (14).

**5.** Dispositif de dialyse (1) selon la revendication 4, **caractérisé en ce que** l'unité de surveillance (11) est conçue pour détecter une disconnexion d'aiguille en tant qu'état de fonctionnement sur la base de la pression différentielle $p_{diffm}$ mesurée et/ou d'une modification de la pression différentielle $p_{diffm}$ mesurée.

**6.** Procédé de surveillance d'un état de fonctionnement d'un dispositif de dialyse (1) qui comprend un système de conduite de liquide (2) rempli d'un liquide autre que le sang avec une première section (3) et une deuxième section (4), comprenant les étapes suivantes

- mesure (S1) d'une pression différentielle $p_{diffm}$ entre une première pression ($p_1$) dans la première section (3) et une deuxième pression ($p_2$) dans la deuxième section (4) au moyen d'un capteur de pression différentielle (6),
- détermination (S2) de l'état de fonctionnement sur la base de la pression différentielle $p_{diffm}$ mesurée, et
- émission d'un message sur la base de l'état de fonctionnement déterminé,
**caractérisé en ce que** le procédé comprend les étapes suivantes
- mesure (S4) d'une pression ($p_1$) dans la première section (3) au moyen d'un premier capteur de pression (7),
- mesure (S5) d'une pression ($p_2$) dans la deuxième section (4) au moyen d'un deuxième capteur de pression (8),
- calcul (S6) d'une pression différentielle $p_{diffb}$ entre la pression mesurée ($p_1$) dans la première section (3) et la pression mesurée ($p_2$) dans la deuxième section (4),
- détermination (S7) de l'état de fonctionnement sur la base de la pression différentielle $p_{diffb}$ calculée et de la pression différentielle $p_{diffm}$ mesurée,

dans lequel le procédé est réalisé avant et/ou après un traitement.

**7.** Procédé selon la revendication 6, **caractérisé en ce que** l'état de fonctionnement comprend une fonction incorrecte du premier et/ou du deuxième capteur de pression (7, 8).

**8.** Procédé selon l'une quelconque des revendications 6 ou 7, **caractérisé en ce que** la détermination de l'état de fonctionnement comprend les étapes suivantes :

- calcul (S8) d'une pression $p_{res}$ résultante à partir de la différence entre la pression différentielle $p_{diffb}$ calculée et la pression différentielle $p_{diffm}$ mesurée, et
- analyse (S9) du comportement de la pression $p_{res}$.

**9.** Procédé selon la revendication 8, **caractérisé en ce que** l'analyse (S9) est une comparaison avec une valeur de consigne et/ou une analyse de la modification dans le temps.

**10.** Procédé selon la revendication 9, **caractérisé en ce qu'**en cas d'écart de la pression $p_{res}$ résultante par rapport à la valeur de consigne, un volume d'erreur est cumulé à partir de la pression $p_{res}$ résultante et en cas de dépassement d'un volume d'erreur maximal prédéfini, le message est émis.

**11.** Procédé selon l'une quelconque des revendications 9 ou 10, **caractérisé en ce qu'**en cas d'écart persistant de la pression $p_{res}$ résultante par rapport à la valeur de consigne, le message est émis lorsqu'un seuil de déclenchement est atteint.

**12.** Procédé selon l'une quelconque des revendications 7 à 11, **caractérisé en ce qu'**après la détermination d'une fonction incorrecte d'un premier capteur de pression (7) dans une première section (3), la pression (p1) dans la première section (3) est déterminée à partir de la pression différentielle $p_{diffm}$ mesurée et de la pression ($p_2$) mesurée dans une deuxième section (4).

Fig. 1

Fig. 2

Fig. 3

Fig. 4

EP 3 773 788 B1

Fig. 5

EP 3 773 788 B1

Fig. 6

24

Fig. 7

Fig. 8

Fig. 9

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 9925323 B2
- US 6601432 B1